Europäisches Patentamt

(19) European Patent Office

Office européen des brevets

(11) Publication number: **0 177 252**
A2

# (12) EUROPEAN PATENT APPLICATION

(21) Application number: 85306792.4

(22) Date of filing: 24.09.85

(51) Int. Cl.⁴: **A 61 B 17/28**
**A 61 B 17/30**

(30) Priority: 26.09.84 IL 73078

(43) Date of publication of application:
09.04.86 Bulletin 86/15

(84) Designated Contracting States:
AT BE CH DE FR GB IT LI LU NL SE

(71) Applicant: Porat, Michael
52 Hamitnadev Street
Tel Aviv 69690(IL)

(71) Applicant: Porat, Amir
22 Rachvat Eilan Street
Givat Shaul Ramat Eilan(IL)

(72) Inventor: Porat, Michael
52 Hamitnadev Street
Tel Aviv 69690(IL)

(72) Inventor: Porat, Amir
22 Rachvat Eilan Street
Givat Shaul Ramat Eilan(IL)

(74) Representative: Fisher, Bernard et al,
Raworth, Moss & Cook 36 Sydenham Road
Croydon Surrey CR0 2EF(GB)

(54) Medical Forceps and like medical instruments.

(57) A forceps or like medical instrument having a core (1, 22, 22') of metal, its gripping members (3, 4, 21, 22, 21', 22') being at least partially covered by a molded-on layer of plastics in a sandwich-like manner. In particular, a forceps or like medical instrument wherein the part of the core which is destined to become the gripping part (5, 24a, 24a') is covered by a plastic gripping means with or without teeth (12, 24, 24'). Desirably, the gripping members (3, 4, 21, 22, 21', 22') are of substantially identical construction.

EP 0 177 252 A2

./...

Croydon Printing Company Ltd

FIG.1

## Medical Forceps and Like Medical Instruments

The present invention concerns forceps and like medical instruments such as clamps, tongs, tweezers and pincers used in medicine, hereinafter collectively referred to as "forceps".

Forceps made of metal are known and their manufacture is generally costly since the part which performs the gripping action has to be finished by hand. If these forceps are mass-produced their quality is generally inferior especially as far as accuracy of the gripping part is concerned, since this gripping part must contain grooves and teeth in order to be able to grip veins, delicate threads, hair, body tissue and the like.

There are known plastics forceps which have been produced to overcome the disadvantages of the metal ones, since the teeth and grooves of the gripping parts can be manufactured accurately by molding. However, these forceps are not very strong and are liable to bend during use.

There are also known scissors in which the cutting part is made of metal, while the handles are either of plastics entirely or are of metal coated with plastics. The plastics coating here is for convenience only. There are furthermore known medical scissors-like clamps in which the finger-grips only are made of plastics, the metal gripping parts being inserted therein.

The present invention seeks to provide a forceps or like medical instrument which combines the advantages of both metal and plastics ones i.e. the elasticity of metal and the ability of plastics to achieve high accuracy when molded.

The invention consists of a medical forceps or like medical instrument having a core of metal, characterised in that its gripping members are at least partially covered by a molded-on layer of plastics in a sandwich-like manner.

The invention is illustrated by way of example only in the accompanying drawings in which:

Figure 1 is a plan view of a medical forceps according to the invention,

Figure 2 is a side elevation thereof,

Figure 3 is a section taken on line III-III of Fig. 1,

Figure 4 is a section taken on line IV-IV of Fig. 3,

Figure 5 is a plan view of a medical clamp according to the invention in the open position, and

Figure 6 is a similar view of another embodiment thereof.

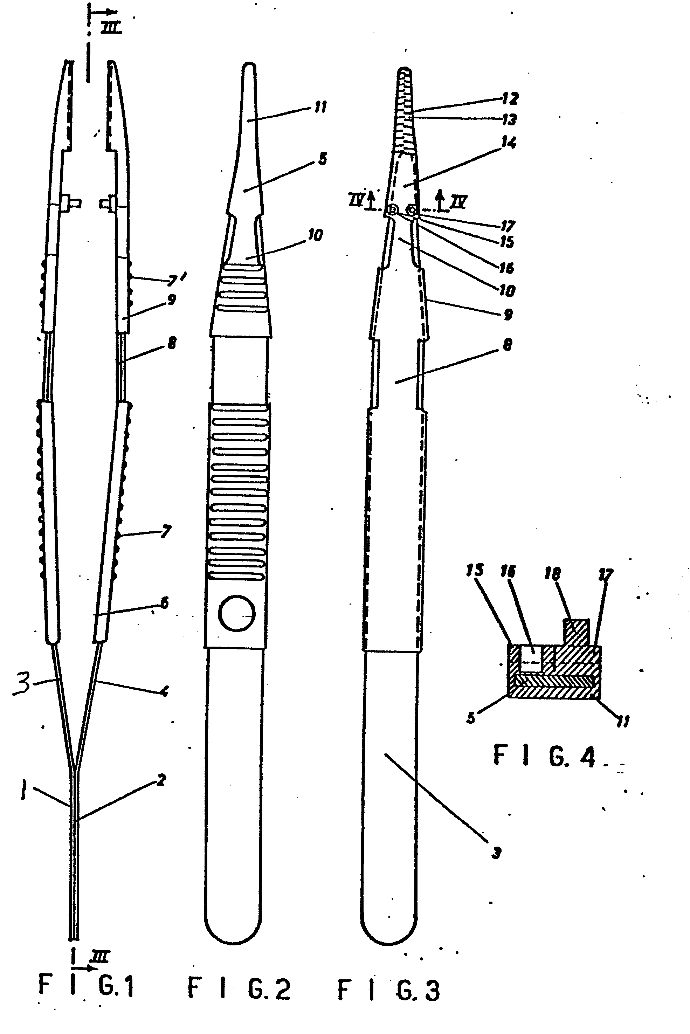

The forceps illustrated in Figs. 1 to 4 comprises a metal core 1 in the shape of known forceps, i.e. the handle part 2 and the gripping members 3 and 4, having the gripping part 5 which is without the teeth and grooves. The gripping members 3 and 4 are covered in a sandwich-like manner by a plastics molded-on covering which envelopes them at a first region 6 close to the handle part 2, the region 6 being smooth on the inside and having ribs 7 on the outside to facilitate grasping when the forceps are held in the hand. A first bridge member 8 merges with region 6 only on the inside of the gripping member, a second region 9 having ribs 7' on the outside merging with the bridge member 8 and likewise surrounding the core 1. A second bridge member 10 merges with region 9 both on the inside and the outside. The gripping portion 11, which is of increased thickness and envelopes the gripping part 5, merges with bridge member 10 and is smooth on the outside and has teeth 12 and grooves 13 on the inside. The teeth 12 and grooves 13 are arranged in two groups and lie at the same angle on either side of an imaginary longitudinal median line 14. The row of teeth 12 which lies on one side of line 14 is staggered in relationship to those on the other side of said line, as described and claimed in our co-pending patent application of even date filed under Agent's reference 29633. By this means, a substantial mating fit is provided between the teeth and grooves of the gripping portion of one gripping member with the grooves and teeth of the gripping portion of the other gripping member when the two gripping members are brought together in use.

Adjacent second bridge member 10 guide means are provided which comprise on one side of line 14 first integral cylinder 15 in which a depression 16 is provided and on the other side of said line 14 second integral cylinder 17 away from which a short pin 18 extends, the diameter of pin 18 corresponding substantially to that of depression 16. Thus when the two gripping members 3, 4 of the forceps face each other, on each side of line 14, the teeth 12 of one row will engage in the grooves 13 of the other row and pin 18 will engage in depression 16 of the opposite gripping member. Owing to this construction, forceps having strength and excellent accuracy are provided while the elasticity of the metal is retained to provide a good gripping action.

It can be seen that the forceps can be mass-produced by making two identical parts which can be welded together at the handle part 2, since it is without a plastics covering. In this manner the costs of the forceps are minimal. This is particularly important where the forceps are "disposable", i.e. intended to be used only once.

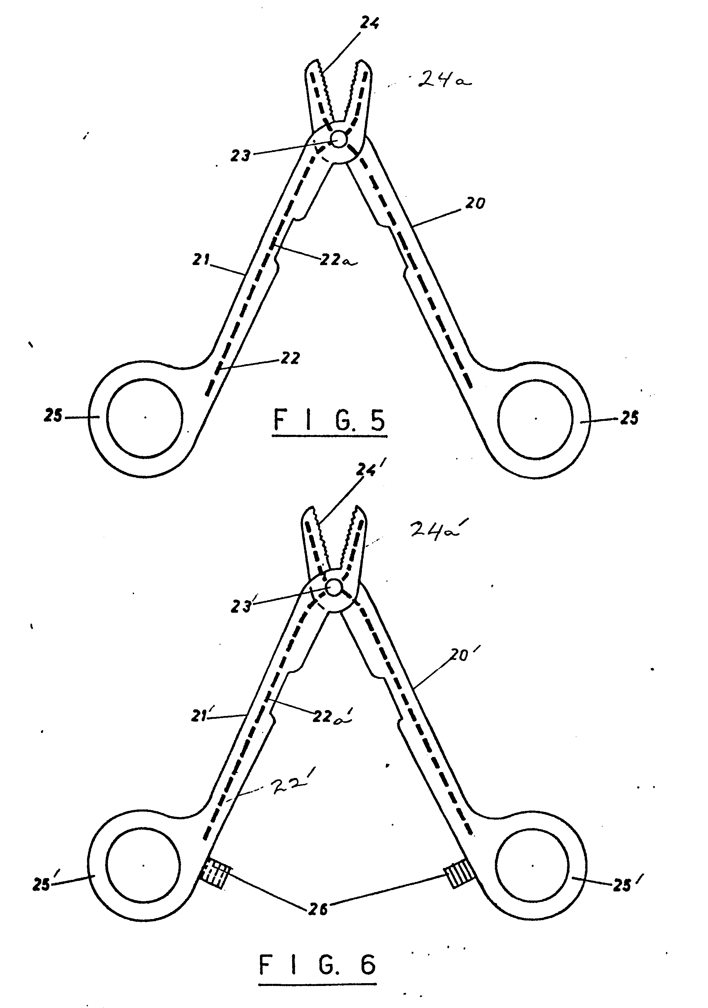

The medical clamp shown in Fig. 5 is of scissors-like shape, the identical gripping members 20 and 21 having a core 22 of metal (indicated in dashed lines) and being covered by plastics in a sandwich-like manner except for region 22a', the members being pivotally connected to each other by a rivet 23 of metal or plastics. The gripping teeth 24 of gripping part 24a are similar to those described with reference to Figs. 1 to 3. The finger-engaging part 25 of the gripping members, 20, 21 are here entirely of plastics. The core 22 may, however, be extended to form a core for the finger-engaging part 25.

The medical clamp shown in Fig. 6 is similar to that of Fig. 5 and the same parts are denoted by the same reference numerals but primed. The only difference is that parts 25 are each made integral with one half of a locking member 26, which inter-engage to lock together when the clamp is closed.

It can be seen that these clamps, like the above-described forceps, can be accurately mass produced and easily connected to each other.

If desired, the plastics covering may be over the entire core of the forceps or clamps, or may be over a part of the core which is less than that described above. Furthermore, the forceps and the clamps may be provided with a gripping part without teeth and grooves.

CLAIMS:

1. A medical forceps or like medical instrument having a core of metal (1, 22, 22'), characterised in that its gripping members (3, 4, 21, 22, 21', 22') are at least partially covered by a molded-on layer of plastics in a sandwich-like manner.

2. A medical forceps or like medical instrument as claimed in claim 1, characterised in that the part of the core which is destined to become the gripping part (5, 24a, 24a') is covered by a plastics gripping means.

3. A medical forceps or like medical instrument as claimed in claim 2, characterised in that the plastics gripping means defines teeth (12, 24, 24').

4. A medical forceps or like medical instrument as claimed in any one of the preceding claims, characterised in that guide means are provided on the plastic gripping members (3, 4) on both sides of a longitudinal median line (14), comprising on one side of said line a first cylinder (15) having a depression (16) and on the other a second cylinder (17) from which a pin (18) extends, the diameter of the pin (18) corresponding substantially to that of the depression (16).

5. A medical forceps or like medical instrument as claimed in any one of the preceding claims, characterised in that each gripping member (3, 4) is made separately, and two such members are welded together at their handle parts (2) which are bare of a plastics covering.

6. A medical forceps or like medical instrument as claimed in any one of claims 1 to 4, characterised in that each gripping member (21, 22, 21', 22') is made separately, their parts being hingedly connected by a rivet (23, 23').

-6-

0177252

7. A medical forceps or like medical instrument as claimed in claim 6, characterised in that locking members (26) are provided near the handle part (25').

8. A medical forceps or like medical instrument as claimed in any one of the preceding claims, characterised in that the parts (25, 25') destined to be held in the hand are of plastics.

9. A medical forceps or like medical instrument as claimed in any one of claims 1 to 7, characterised in that the parts (6) destined to be held in the hand are of plastic-covered metal.

10. A medical forceps or like medical instrument as claimed in any one of the preceding claims, characterised in that its gripping members (3, 4, 21, 22, 21', 22') are of substantially identical construction.

F I G.1　F I G.2　F I G.3

F I G. 4

0177252

F I G. 5

F I G. 6